# EUROPEAN PATENT SPECIFICATION

(11) **EP 4 377 408 B1**
(45) Date of publication and mention of the grant of the patent: **08.04.2026**
(21) Application number: 22726294.6
(22) Date of filing: 03.05.2022
(51) Int. Cl.: C09J 123/02, C08L 23/20, C09J 123/20, A61F 13/02, A61F 13/15

(54) **HOT-MELT ADHESIVE FORMULATIONS WITH IMPROVED PROCESSABILITY IN SLOT-DIE COATING**
SCHMELZKLEBSTOFFFORMULIERUNGEN MIT VERBESSERTER VERARBEITBARKEIT IN DER SCHLITZDÜSENBESCHICHTUNG
FORMULATIONS ADHÉSIVES THERMOFUSIBLES À APTITUDE AU TRAITEMENT AMÉLIORÉE EN REVÊTEMENT PAR FILIÈRE DROITE PLATE

(30) Priority: 27.07.2021 IT 202100020009
(43) Date of publication of application: 05.06.2024
(73) Proprietor: Savare' I.c. S.r.l., 20156 Milano (IT)
(72) Inventor: BUGANA, Alberto, Milan (IT); BRIATICO VANGOSA, Francesco, Milan (IT)
(74) Representative: Vatti, Francesco Paolo
(86) International application number: PCT/IB2022/054076
(87) International publication number: WO 2023/007257

(56) References cited:
- WO-A1-2017/066266
- US-A1- 2018 282 451
- US-A1- 2021 115 232

## Description

### FIELD OF INVENTION AND TECHNICAL BACKGROUND

The present invention discloses hot-melt adhesive formulations that, besides presenting excellent properties in use, both for what concerns adhesiveness and cohesion, show also an unexpected and strongly improved processability that allows to use them without any problem even on industrial lines operating at high speed, e.g., at 200 m/minute, both in spraying and in slot-die coating.

As it is well known, hot-melt adhesives are polymeric blends that comprise, as their main component(s), one or more thermoplastic polymers, together with variable quantities, for example from zero to even 90% by weight, of other optional components and additives, like especially plasticisers and tackifiers.

Nevertheless, it is unquestionable that the nature and the features of the polymer (or polymers) present in the adhesive formulation, play the most important role in determining all the major properties of the final adhesive, even in those cases in which the polymer or the blend of polymers form just a fraction, even smaller than 50% by weight, of the total adhesive composition. Moreover, it is also well known that hot-melt adhesive formulations are processed in the molten state, generally at temperatures from about 130°C and 180°C. by using essentially two types of application process:
- Spraying/Fiberisation processes, in which the molten adhesive's flow is extruded through a nozzle, inside which the flow is hit by a strong stream of hot air. This air divides the molten adhesive's flow into fine particles that, based on the geometrical structure of the nozzle, can have different shapes and sizes, like fibrils, droplets more or less spherical or elongated etc.
- Slot-Die coating processes, in which the molten adhesive's flow is extruded, in the form of a continuous film, through a metallic head/die that has a substantially rectangular shape and that has one or more hole(s), most often rectangular and horizontal, these holes being generally a few tenths of millimeter high and a few centimeters wide.

Both these processes, and especially the slot-die coating process, are not specific just for hot-melt adhesives, but they are also commonly applied to the process of pure polymers or of polymeric blends also for other uses.

As for example, in the article "Historical Review of Die-drool phenomenon during Plastics Extrusion" by J. Musil e M. Zatloukal, published in AIP (American Institute of Physics) Conference Proceedings, 1526 (16) - 2013 - pages from 16 to 34, several polymers and their blends and formulations, included hot-melt adhesive formulations, show severe problems in extrusion, in particular in die-slot coating/extrusion, problems that may make extremely difficult or even impossible the application; and that anyhow always worsen the quality and the properties of the final result, because they cause strong unevennesses and inhomogeneities in the extruded film of the polymer or of the polymeric composition.

In particular, the most important and most deleterious of such extrusion problems, that prevent the formation of a regular and homogeneous film of extruded adhesive, is the phenomenon known with the terms "die-drool" or "die build-up". This phenomenon, as in details explained in the above mentioned article, consists in that a part of the extruded material/polymeric formulation remains adhered on the metal of the extrusion-head itself, and builds up in masses of increasing weight and volume, on the metallic surface just outside and around the extrusion holes.

Said masses of built up material, that in the meanwhile also start to degrade owing to the high temperature on long times, casually and irregularly come off from the extrusion-head, stick on the substrate that takes these lumps of material away, while the build-up process restarts. In this way the quality of the finished product is irretrievably spoiled, often at unacceptable levels, by this substantially chaotic and uncontrollable phenomenon, both because the coating is a grossly irregular film with uneven thickness, and because of the presence at random gaps of big lumps of adhesive, that moreover are also strongly degraded.

The previous Art tried to solve this grave problem of die-drool/die build-up, in various ways; but however none of them is fully satisfactory. For example, WO 2020139703, by Stuczynski et.al., for avoiding such a problem, teaches to increase the temperature of the whole extrusion die system, from a minimum of 5°C to a maximum of 60°C over the temperature at which the two rheological moduli of the material, G' and G", cross, temperature that is also known as the "Rheological Setting Temperature" of the material. However, it is apparent that such a method does not teach at all a general solution for the build-up problem, because generally the temperatures, in the extrusion of a hot-melt adhesive, cannot first of all be freely changed, even more in such a wide range like about 60°C; this because it is necessary to keep in mind that in many of the most important uses of hot-melt adhesives, like the manufacturing of hygienic absorbent articles (a use that by the way WO 2020139703 claims as a "particularly preferred use", see page 4, lines 15-18) substrates like plastic films and plastic nonwovens are used, that are highly thermosensitive, and that would be deformed, partially melted or even burned by the contact with metallic parts, like rolls, that are too hot, or by the contact with an adhesive extruded at too high temperatures. Moreover, our experiments have ascertained that, for several polymeric formulations, an increase in the extrusion temperature can even worsen said negative phenomenon of die build-up. Furthermore, it is significant to notice that the patent US 6245271 by Jacobs et al., claims just a positive effect on the die build-up phenomenon of a decrease in the temperature of the apparatus/machine, and not of an increase in temperature. This patent US 6245271 additionally claims beneficial effects on the die build-up phenomenon by peculiar geometries and mechanical designs of the extrusion heads, in this statement followed also by other patents like US 6358449 di Tinsley et al.

It is apparent that because the optimum geometry of extrusion dies for avoiding the build-up varies in turn as a function of the extruded material's nature and characteristics, said solution cannot be easily applied, in a practical and economical way, on industrial lines that process hot-melt adhesives the nature of which changes frequently according to the different articles manufactured on those lines. The use of non-standard extrusion dies, designed and "tailor-made" and moreover changed every time that a line changes the used adhesive, would lead to unacceptable costs, both in terms of investments as well as of exercise of said line.

Finally, another group of patents, for example US 4904735 ed US 5089200 by Chapman et al., US 5710217 by Blong et al., WO 2107066266 by Lavallee et.al., teach to solve the build-up problem by the addition, inside the extruded thermoplastic material, of very small quantities of special additives, in particular fluorinated polymers, that can act as lubricants during the extrusion of the molten polymeric composition, preventing its adhesion on the metallic surfaces and therefore preventing the build-up. However, it is manifest that this solution for the build-up problem, by the addition of fluorinated additives that, as everybody knows, have a very powerful anti-adhesive action, cannot be applied, for obvious reasons, to the process of application of hot-melt adhesives, whose adhesive properties would be totally or anyhow significantly destroyed by such additives.

We can therefore conclude that Prior Art does not disclose any fully satisfactory and sufficiently general solutions to the die build-up / die drool problem, solutions that teach how to avoid such problem in a simple, cheap and effective way, without spoiling their adhesive properties and without further costs or additional complications.

WO 2017/066 266 discloses a polymer composition, including a thermoplastic polymer combined with a minor amount of a fluorinated polymer.

US 2018/0 282 451 discloses amorphous propylene-ethylene copolymers, which can include high amounts of ethylene and exhibit desirable softening points and needle penetration, providing them with a broad operating window.

US 2021/0 115 232 discloses a composition comprising a propylene/ethylene copolymer with a density lower than 0.880 g/cc and a MWD from 2.00 to 3.00 and a melt viscosity at 177 °C below 80,000 mPa.s with an olefin multi-block copolymer exhibiting a density lower than 0.890 g/cc and a melt index higher than 0.5 g/10 min.

### SUMMARY OF THE INVENTION

The problem that the present invention aims to solve is to disclose how to formulate hot-melt adhesives that do not present during extrusion, and especially in slot-die coating, the highly negative phenomenon known as "die drool" or "die build-up" and other similar phenomena that cause an irregular and inhomogeneous flow in extrusion.

Said hot-melt adhesives according to the present invention have, on the contrary, an optimum processability, even improved, also on industrial lines operating at high speed (e.g. 200 m/minute and above) still using standard extrusion heads / dies and without varying too much the fundamental process parameters, like the extrusion apparatus temperature, keeping such temperature at its optimum level, typical of a certain adhesive, temperature that, if varied too much might cause further problems like damages of thermosensitive substrates, if temperature is too much increased, or possible problems of excessive viscosity, if this temperature is decreased.

This significantly improved processability in extrusion, especially without phenomena of build-up or other similar phenomena that lead to an irregular and inhomogeneous extrusion, is achieved in particular by choosing the polymer or the blend of compatible polymers, forming the fundamental component of the hot-melt adhesive, in such a way that said polymer or blend of polymers pass the experimental test called "Induced crystallisation under shear-stress" that is explained in details below, provided that such polymer or blend of polymers have also a Poly-dispersity Index, Mw/Mn, greater than 2.0 and preferably greater than 2.5. It has been furthermore found that, in order to further optimise their processability, the hot-melt adhesive formulations according to the present invention, at the same time should present also the following physical and rheological properties, that can additionally affect the process, especially a slot-die coating process:
- a Brookfield viscosity at 170°C, measured according to the Method ASTM D 3236 - 88, comprised between 500 mPa.s and 50,000 mPa.sa Ring & Ball softening temperature, measured according to the Method ASTM D 36 - 95. not higher than 150°C
- an Elastic Modulus G', measured at 23°C at the frequency of 1 Hz, not lower than 0.05 MPa.

These problems in the application by slot-die coating, e.g. in the manufacturing of hygienic absorbent articles, and when using said adhesive formulations and said manufacturing processes, are solved by a hot-melt adhesive formulation that has the features of claim 1) and of all the claims from 2) to 21; by a glued structure has the features of claim 22); and by articles that have the features of claims from 23) to 30). The other subclaims disclose preferred embodiments.

### DEFINITIONS

The expressions "comprising" or "that comprise(s)" are used herein as open-ended terms, that specify the presence of what in the text follows said terms, but that does not preclude the presence of other ingredients or features, e.g. elements, steps, components, either known in the art or disclosed herein.

The expression "copolymer(s)" is used herein to mean a polymer in the chemical composition of which at least two monomers or more than two monomers are present. Therefore, the term "copolymer(s)" means herein, unless the contrary is specifically stated, not only polymers in the chemical composition of which two different monomers are present, but also polymers in the chemical composition of which three, four, five or more different monomers are present.

The equivalent expressions "Rheological Setting Point" or "Rheological Setting Temperature" or "Temperature of the Crossing of the Moduli" or also "Crossover Point" and its symbol Tx, mean, in a rheological diagram in which they are measured, as a function of temperature, the Elastic Modulus G', the Viscous Modulus G" and their ratio Tan Delta, the temperature at which the two Moduli cross (and in which therefore the value of Tan Delta is equal to 1) in the field of temperatures above room temperature. Said rheological diagram, when measured in decreasing temperature, with a sufficiently slow cooling rate (e.g. 2°C/minute, as done herein), mimics very well the phenomena that occur between the adhesive and a substrate in the real process of the application of a hot-melt adhesive from the molten state and of the subsequent creation of the adhesive bond, during the slow spontaneous cooling and setting/solidification. The "Rheological Setting Point" Tx identifies in particular the temperature at which the hot-melt adhesive, when applied in the molten state on a substrate, starts forming the final adhesive bond in the solid state.

The expression "Crossover Modulus" and its symbol Gc mean, again in the above mentioned rheological diagram, the absolute value (that by definition is identical), expressed in Pa or in MPa, that the Elastic Modulus and the Viscous Modulus have at the "Rheological Setting Point" Tx.

"Room temperature", if not specifically defined in a different way, means a temperature equal to 23 °C; and "room conditions" means the conditions of an environment at a controlled temperature and relative humidity, at 23 °C and 50% relative humidity.

If not specifically defined in a different way, all the rheological parameters mentioned in the present invention, e.g., the Elastic Modulus G' of a certain material, are measured at a frequency of 1 Hz, in a rheological test under conditions of decreasing temperature, between 170°C and - 20°C, at a temperature decreasing rate of 2°C/minute.

"Hygienic absorbent articles" refer to devices and/or methods concerning disposable absorbing and non-absorbing articles, that comprise diapers and undergarments for incontinent adults, baby diapers and bibs, training pants, infant and toddler care wipes, feminine catamenial pads, interlabial pads, panty liners, pessaries, sanitary napkins, tampons and tampon applicators, wound dressing products, absorbent care mats, detergent wipes, and the like.

The expression "compatibility" and the adjective "compatible", referred to the mutual blends of the ingredients of the present hot-melt adhesive formulations, and in particular to the blends of two or more polymers, are herein considered in the meaning defined in the "IUPAC. Compendium of Chemical Terminology"-2nd Edition- 1997. i.e., a blend is "compatible" when it shows macroscopically uniform physical properties, independently of the fact that it is formed by "miscible" blends (i.e. that present just one Glass Transition Temperature, Tg) or by "immiscible" blends (i.e. with two or more Tg's). In particular, the present invention considers as "compatible" all those blends that, when kept in the molten state at 170°C for 72 hours, do not show any visual de-mixing in two or more layers / phases.

"Polydispersity Index" or "Molecular Weights Distribution Index" or "PDI" refers to a measure of the distribution of the molecular weight in a certain polymer. It is defined as the ratio between the weight average molecular weight Mw, and the number average molecular weight Mn: PDI = Mw / Mn. Greater values of PDI correspond to broader distribution curves of molecular weights and vice versa. Even for compatible blends of polymers it is possible to define an average Mw, an average Mn and therefore a global "Index of Polydispersity" as defined in the case of single polymers. Mw, Mn and their ratio Mw / Mn = PDI, are measurable e.g., by Gel Permeation Chromatography (GPC).

"Open Time" of an adhesive refers, especially for a hot-melt adhesive, to the interval of time during which, after its application from the melt on a first substrate, the adhesive is able to form sufficiently strong adhesive bonds for the intended use, with a second substrate that is brought into contact under moderate pressure with the first one. It is apparent that too short open times may make difficult-to-manage the application of an adhesive and the formation of sufficiently strong bonds. The open time of a holt-melt adhesive may be measured according to the test method ASTM D 4497 - 94, with the following conditions for the hot-melt adhesives disclosed herein:
- Coating temperature of the adhesive film: 170°C
- Thickness of the adhesive film: 1 mm

"Ring & Ball Softening Point" or "Ring & Ball Softening Temperature" refers to the softening temperature of a material, measured according to the Method ASTM D 36 - 95.

Just for waxes, the Softening Point (known in this case also as "Dropping point") is measured according to the Method ASTM D 3954 - 94.

The "Needle Penetration" of an adhesive is a measure of its softness. It is generally expressed as tenths of millimetre, dmm, and it is herein measured at 55°C, according to the Method ASTM D1321-04.

The dynamic viscosity of a molten or liquid material at a certain temperature is expressed as mPa.s and it is measured according to the Method ASTM D 3236 - 88.

The overall Adhesive Strength or "Peel Strength" is defined as the average strength per unit of width needed to separate two substrates, bonded by the adhesive under test, measured through a separation test made at a controlled and constant speed, and under a controlled and constant debonding angle. It is herein measured according to the Method ASTM D 1876 - 01, separating the two substrates under a debonding angle of 180 degrees, by applying a separation speed of the two substrates equal to 150 mm/minute, that means that the testing dynamometer is actually moving at a speed of 300 mm/minute. The two substrates used herein are a microporous polyethylene film, with a basis weight of 22 g/m², on which the molten adhesive is directly applied, by spraying or by slot-die extrusion, and on which a spunbonded polypropylene nonwoven with a basis weight of 12 g/m² is immediately bonded. The measurement of the Peel Strength is made by recording the average strength needed to separate the two bonded substrates on a width of 50 mm.

The "Tensile Propertiers" or "Mechanical Properties in Tension" of all the materials disclosed herein, e.g. the hot-melt formulations, like the so called "Stress - Strain Curve", and the respective "Peak Stress" or "Ultimate Tensile Strength"; the "Stress at Break"; and "Elongation at Break" etc., are herein measured at 23°C and 50% Relative Humidity, according to the following method. For each adhesive under test, five rectangular samples are prepared, by casting the molten adhesive at 170°C into a silicone mould. Each rectangular sample has a length of 25 mm, a width of 6 mm and a thickness of 2 mm. After their solidification, the samples are taken out of the mould and are aged for five days at 23°C and 50% Relative Humidity. For assessing its Stress - Strain curve to break, each sample is fixed with clamps, at its narrowest ends, to the shafts of a Rheometer Ares G2, available from TA Instruments (or any other similar rotational rheometer, having a suitable system for temperature control and for rapid cooling) equipped for Tensile tests with the so called "Torsion Rectangular" geometry. The free length of the sample is 10 mm. The mobile upper shaft, that has a maximum further run of 60 mm (hence corresponding to a maximum readable strain of 600%), is started at an extension rate equal to 6 mm/minute.

These test conditions hence correspond to a Strain Rate of the sample equal to 0.01 s⁻¹, i.e. a frequency of 0.01 Hz, that, as well known to every person averagely expert in the Rheology of adhesives, is the typical frequency at which said phenomena of slow detachment under load occur.

The tensile test is stopped either when the sample is fractured or when the rheometer has reached the maximum readable strain equal to 600%. For each Stress - Strain to Break curve, the rheometer measures the maximum "Peak Stress" or "Ultimate Tensile Strength", the maximum Strain and it also calculates the area under the curve itself, area that is equal to the Toughness of the material, expressed in J/m³.

Other less usual parameters, that are measured according to specific methods, will be defined later, together with the detailed description of the methods for measuring them.

### DETAILED DESCRIPTION OF THE PREFERRED EMBODIMENTS AND MAIN FEATURES OF THE ADHESIVES ACCORDING TO THE PRESENT INVENTION

The hot-melt adhesive formulations according to the present invention show an optimum processability, even strongly improved versus the status of the Prior Art, In particular, they can be very easily processed and applied in the slot-die coating process even at high and very high speed, like for example 200 m/minute and over, without especially showing the very grave problem called "die drool" or also "die build-up".

This surprising and very advantageous property is achieved by formulating the present hot-melt adhesive formulations according to the criteria and by using the particular raw materials described below.

In particular, the hot-melt adhesive formulations according to the present invention comprise, as their fundamental component, a polymer (that may be both a homopolymer or a copolymer) or a blend of two or more mutually compatible polymers (that may be both homopolymers or copolymers) as defined in claim 1 that show, at the same time of measure a maximum difference - according to the below defined test of "Induced crystallization under shear-stress" - between the Elastic Modulus G' at 80°C, measured after the pre-application of a certain shear-stress, and the Elastic Modulus G' at 80°C, measured without the pre-application of any shear-stress, that is not greater than 100%. Such an experimental result can be also conveniently defined by saying that, in said test, the maximum value of the ratio between the Elastic Modulus G' at 80°C, after the pre-application of a shear-stress, and the Elastic Modulus G' at 80°C, without any pre-application of a shear-stress, is not greater than 2.0.

Said polymer or blend of compatible polymers, that do not show a difference that is greater than 100% between their Elastic Moduli G', measured with and without the pre-application of a certain shear-stress according to the method described in more detail below, and that have a global Polydispersity Index greater than 2.0 and preferably greater than 2.5, are comprised in the present hot-melt adhesive formulations in a quantity from 10% by weight to 99.5% by weight of such hot-melt adhesives , and preferably from 20% by weight to 90% by weight.

We have also found that, in order to further optimise their processability, it is opportune that the hot-melt adhesive formulations according to the present invention have at the same time also the following rheological and physical properties, that may affect the process, in particular a slot-die coating process:
- a Brookfield viscosity at 170°C, measured according to the Method ASTM D 3236. 88, ranging between 500 mPa.s and 50,000 mPa.sa Ring & Ball softening temperature, measured according to the Method ASTM D 36 - 95.not higher than 150°C
- an Elastic Modulus G', measured at 23°C at the frequency of 1 Hz, not lower than 0.05 MPa.

### Other components of the hot-melt adhesives according to the present invention

### Tackifiers

In an embodiment of the present invention, the adhesive compositions of the present invention also comprise at least one tackifying resin, having a Ring & Ball softening point, measured according to the Method ASTM D 36 - 95, ranging between 5°C and 160°C.

In general, the tackifiers included in the formulations of the present invention can be selected among the aliphatic hydrocarbon tackifiers, and their partially or fully hydrogenated derivatives; the aromatic hydrocarbon tackifiers, and their partially or fully hydrogenated derivatives; the aliphatic/aromatic tackifiers, and their partially or fully hydrogenated derivatives; the terpenic tackifiers, and their partially or fully hydrogenated derivatives; the rosins, their esters and their partially or fully hydrogenated derivatives. Fully hydrogenated hydrocarbon tackifiers, both aliphatic and aromatic and aliphatic/aromatic, are particularly preferred.

Furthermore, it has been discovered that it is preferable that the tackifying resins, used in the formulations of the present invention, have a Ring & Ball softening temperature, measured according to the Method ASTM D 36 - 95, ranging between 70°C and 135°C, preferably between 80°C and 130° and more preferably between 85°C and 125°C.

Particularly preferred tackifiers in the present invention are highly purified tackifiers that contain extremely low quantities of impurities and monomeric volatile components, like xylene, toluene, hexane, vynil-toluene, indene, etc. that contribute to generate malodours in the final finished product and to decrease the thermal stability of the tackifying resin. Said volatile compounds are measured by ionic Gas-Chromatography with head-space by heating a 2 grams samples of the resin for 30 minutes at 190°C with a head-space equal to 20 ml. The tackifiers containing a level of volatiles not greater than 5 ppm (parts per million), preferably not greater than 2 ppm and more preferably non greater than 1 ppm are particularly preferred in the present invention. Industrial examples of said tackifiers having a very low content of volatile impurities are e.g. the tackifiers supplied by Eastman (USA) under the trade mark UltraPure.

In the embodiment of the present invention in which the hot-melt adhesive formulations comprise at least one tackifying resin, they include zero to 75% by weight of at least one tackifier or of a blend of tackifiers, preferably between 10% by weight and 70% by weight, and more preferably between 20% by weight and 65% by weight referred to the total hot-melt adhesive formulation.

### Plasticisers

In another embodiment of the present invention, the herein disclosed hot-melt adhesive formulations include at least one plasticiser, that is liquid at room temperature, i.e. at 23°C, or a liquid blend at room temperature of two or more plasticisers. These plasticisers further lower the melt viscosities of the adhesive formulations and increase their tackiness. Plasticisers that can be used in the hot-melt adhesive formulations according to the present invention, are selected for example from paraffinic mineral oils, naphthenic mineral oils and their blends; paraffinic and naphthenic hydrocarbons that are liquid at room temperature, and their blends; oligomers, that are liquid at room temperature, of polyolefins and of their copolymers, like liquid oligomers derived from ethylene, propylene, butene, iso-butylene, their copolymers and the like, and their blends; liquid plasticisers formed by esters, like phthalates, benzoates, sebacates; vegetable oils and their blends; natural and synthetic fats; and their blends. Mineral oils, both paraffinic and naphthenic, and their blends, are particularly preferred. Equally preferred are liquid oligomers of polyolefins, synthesised through metallocene catalysts. Said synthetic oligomeric plasticisers, liquid at room temperature, usable in the present invention and known also with the acronym "PAO" (poly-alpha-olefin), are synthesised from olefins from C2 to C20; have an average Number Molecular Weight Mn from 150 to 15,000 g/mole, preferably from 200 to 10,000 g/mole and even more preferably from 400 to 6,000 g/mole. Said PAO plasticisers are fully saturated and can have a chain structure that is substantially paraffinic, either a linear or branched one, with a Polydispersity Index not greater than 2.0, are manufactured and sold e.g. by Exxon Mobil with the trade marks Spectrasyn and Elevast, by Ineos with the trade mark Durasyn, by Chevron Phillips with the trade mark Synfluid etc.

In the embodiment of the present invention in which the herein disclosed hot-melt adhesive formulations include at least a plasticiser or a blend of plasticisers, liquid at room temperature, they comprise from zero to 50% by weight of a plasticiser or of a blend of plasticisers, preferably from 5% by weight to 40% by weight and more preferably from 10% by weight to 35% by weight referred to the total weight of the hot-melt adhesive formulation.

### Waxes

In a further embodiment of the present invention, the hot-melt adhesive formulations disclosed herein comprise at least one wax or a blend of waxes, natural or synthetic; these wax or waxes have a dropping point, measured according to ASTM D-3954-94 that ranges between 40°C and 170°C. Waxes that can be used in the present invention are for example hydrocarbon synthetic waxes, like paraffinic waxes, and in particular waxes synthesised from olefins from C2 to C10 and their blends; hydrocarbon waxes from C12 to C40, also in their versions modified with acidic carboxylic or alcoholic groups; copolymeric waxes synthesized from ethylene and maleic anhydride or from propylene and maleic anhydride, microcrystalline waxes, waxes formed by esters of fat acids from C12 to C40; natural waxes, like beeswax, carnauba wax, montan wax and similar, and their blends.

In the embodiment of the present invention in which the herein disclosed hot-melt adhesive formulations include at least one wax or a blend of waxes, they comprise from zero and 20% by weight of said wax or blend of waxes, preferably from zero to 10% by weight and more preferably from zero and 5% by weight referred to the total weight of the hot-melt adhesive formulation.

### Other additional components

The hot-melt adhesive formulations disclosed herein may furthermore include from 0.01% by weight to 10% by weight of at least one stabilizer, like antioxidants, photo-stabilisers anti-UV and their blends. They may additionally comprise up to a maximum of 15% by weight of other optional additional components, like mineral fillers, dyes, perfumes, surfactants, antistatic agents.

### Peculiar Test Methods

### The test for "Induced Crystallization under Shear-Stress"

As already said, the hot-melt adhesive formulations according to the present invention comprise, as their fundamental component, a polymer (that can be a homopolymer or a copolymer) or a blend of two or more mutually compatible polymers (that can also be homopolymers or copolymers) which show, at the same time of measure according to the herein described test called "Induced Crystallisation under Shear-Stress", a maximum difference between their Elastic Modulus G', at a suitable temperature comprised between 40°C and 110°C , measured after the pre-application of a certain shear-stress, and their Elastic Modulus G' at the same suitable temperature comprised between 40°C and 110°C , measured without the pre-application of a shear-stress, that is not greater than 100%.

Said experimental result can be also conveniently expressed by saying that the maximum value of the ratio between the Elastic Modulus G' at a suitable temperature comprised between 40°C and 110°C , measured after the application of a certain shear-stress, and the Elastic Modulus G' at the same suitable temperature comprised between 40°C and 110°C , measured in said test without any shear-stress, is not greater than the value of 2.0.

We define the generic value of time in the curve that expresses the increasing value of G' as a function of time, in the experiment without any shear-stress, as *t(no-shear);* while *t(shear)* is the generic value of time in the corresponding experiment with a pre-application of a certain shear-stress.

The temperatures at which the two tests, with and without the pre-application of a certain shear-stress, are performed on the same material in two tests in parallel, must obviously coincide. And for each material under test this most suitable temperature is chosen, inside the range between 40°C and 110°C, according to the below illustrated criterion, so that in both tests the full crystallisation starts, occurs and completes in a time frame that is not excessively long, for obvious reasons, but that also is not excessively short, which fact would lead to difficulties in detecting and reading a sufficiently large number of experimental points during the experiment. More in details, for a certain material the most suitable temperature for the two tests of crystallisation is selected in the following way: one always starts from the middle temperature of the range from 40°C to 110°C, i.e., one starts from 75°C, by performing at this temperature a test of crystallisation without the pre-application of any shear-stress.

One assesses the total duration of such experiment of crystallisation, between the starting time t(no-shear)₀, at which the crystallisation and the increase of G' start, and the time t(no-shear) _{asympt} at which the Elastic Modulus G' reaches its final maximum asymptotic value G(t no-shear) _{asympt}, at the completion of its crystallisation. Such time frame must be comprised between 300 seconds and 7,200 seconds.

Moreover, one must also verify that, at the starting time t (no-shear)₀, the initial value of the Elastic Modulus G' (t no-shear)₀ must not be greater than the value of Viscous Modulus at the same time, G" (t no-shear)₀, for assuring that the material is still in a state that can be defined as "rheologically liquid".

If these two conditions are satisfied, the chosen temperature can be considered as suitable for performing the test of "Induced Crystallisation under Shear-Stress", because the time frame connected to this temperature allows to read with sufficient clarity and precision all the data-points in the curve of increase vs. time of the Elastic Modulus G', between t(no-shear)₀ and t (no-shear)_{asympt}.

In the event that none or just one of the above two conditions is satisfied, one must change the temperature, increasing or lowering it in steps of 5°C, until both the two above conditions are fulfilled. More precisely this means that if the time t(no-shear) _{asympt} is shorter than 300 seconds or even more if at t(no-shear)₀ the material has already fully crystallized (i.e. if at t(no.shear)₀, G'' is greater than G') it is necessary to increase of 5°C the temperature at which the test is performed, and so on, until a temperature that allows to start and complete the full crystallization in a time frame comprised between 300 seconds and 7,200 seconds is identified. On the contrary, if the time t(no-shear)_{asympt} is longer than 7,200, it is needed to decrease the operating temperature of 5°C, until a temperature is identified that allows to complete the crystallisation and read the final value of G' (t no-shear) _{asympt} in a time frame between 300 seconds and 7,200 seconds. It has been surprisingly found that hot-melt adhesive formulations that comprise, as their fundamental component, a polymer or a compatible blend of two or more polymers that show such a peculiar behaviour in the test described below, exhibit an exceptionally good processability even on high speed lines, operating at 200 m/minute and above. In particular, differently from similar formulations, based on polymers or on polymeric blends that do not pas this test, the present hot-melt adhesive formulations do not exhibit any phenomenon of die-drool/die build-up when processed by slot-die coating, even in the most severe operating conditions, like at high and very high line-speed.

The aforementioned excellent characteristics of processability are further improved and made even easier to achieve if, moreover, the above mentioned polymer or the compatible blend of polymers, comprised in the present hot-melt adhesive formulations, have a Polydispersity Index that is greater than 2.0 and preferably greater than 2.5.

Said in other words, the inventors of the present invention have unexpectedly found that polymers (and their formulations) that, under the effect of a certain shear- stress in the molten state during their extrusion by slot-die coating, significantly anticipate and accelerate their kinetics of crystallisation, show a very severe fault in processability; in particular they show a very detrimental negative phenomenon of die-drool / die build-up on the extrusion-head, as already described and defined.

Without being for this linked to any theory, one can hypothesise a possible correlation between the two phenomena, for example in the following way. As it is well known to every person with at least an average knowledge in the Science of Polymeric Materials, there is a direct correlation between the crystalline content of a semi-crystalline polymer and its Elastic Modulus G'. A significant anticipation and acceleration, during the extrusion, of the crystallisation of the molten polymer that is flowing out from the extrusion-head, caused not only by the spontaneous decrease in temperature but also by an effect caused by the very high shear-stress felt by the flowing molten polymer especially in the latest centimetres before the exit, can cause a substantial and unexpected increase in the natural elasticity of the molten material, in vicinity of the exit-die.

This undesired increase in the elasticity of the molten material can cause gross inhomogeneities and distortions in the just extruded adhesive film. A part of the extruded material tends to adhere on the metal of the extrusion-die itself and to build up, in masses of increasing volume and weight, especially around the extrusion holes, causing in this way very severe problems of "die build-up".

It is also possible to suppose that, beyond the basic capability that most polymers have of crystallising up to a certain defined level of final crystallinity by cooling and solidification from the melt, a few of them (probably owing to peculiar characteristics in their chain-structures) show, still at high and constant temperature, a significant anticipation and acceleration of their crystallisation, caused by the application of a certain shear-stress. One can hypothesise that these shear-stresses, applied on the molten polymer, and that have a specific and well defined direction, can, in certain polymers, align the various polymeric chains, even if the material is still in the molten state, in this way anticipating and making quicker and easier the formation of ordered crystalline structures, independently of the spontaneous crystallisation that occurs for simple solidification in cooling.

In details, a possible anticipation and acceleration in the crystallisation at a constant temperature, induced by the application of a certain shear-stress, is herein measured in the following way: a sample of the polymer or of the compatible polymeric blend under test, is positioned between the two plates of rheometer Ares-G2, supplied by TA Instruments (or of a similar rotational rheometer). The rheometer must have an ETC oven for the heating and a cooling system suitable for a rapid cooling.

The material under test is heated to 180°C and is molten. Then it is conditioned by leaving it at this temperature for 300 seconds. This initial heating and conditioning at 180°C for 300 seconds is mainly aimed at fully erasing any possible "memory" of the sample's thermal history in the material, by bringing the material into its fully amorphous state, destroying all the crystals that have possibly formed in the previous thermal treatments. In this way, every material under test has the same "thermal history" as all the other ones.

The sample is then rapidly cooled, with a cooling rate of 60°C/ minute, to 140°C and is left at this temperature for 300 seconds. It is worthy to notice that 140°C is the average temperature at which most hot-melt adhesive are inside the extrusion-head, immediately before their extrusion.

At this point, for checking whether or not the tested polymer or blend of polymers exhibits a significant anticipation and acceleration of its crystallisation as an effect of an applied shear-stress, two tests in parallel are performed on the same tested material: in one test the material, molten at 140°C is subjected for 600 seconds to a shear-rate of 50 s⁻¹, the shear-stress being generated through the rotation of the mobile plate of the rheometer; and another parallel test in which the same material is kept at the same temperature of 140°C for 600 seconds without applying any shear-stress.

After 600 seconds at 140°C, with and without an imposed shear-stress, for simulating the conditions met after the exit from the die and the following cooling, the shear-stress is in any case stopped and the samples are further rapidly cooled at the highest possible cooling rate allowed by the rheometer, equal to 60°C/minute until the temperature of 80°C is reached. This temperature significantly represents the average temperature at which an extruded hot-melt adhesive formulation is, in about the first five centimetres of distance from the extrusion die, immediately after its extrusion.

When the samples' temperature reaches 80°C, the rheometers, in the two tests in parallel, start to measure and to record the rheological parameters of the tested materials as a function of the test-time and in isothermal conditions. This measure occurs in an oscillatory regime at the frequency of 1 Hz and under a strain of 0.1%.

In particular, during this latest phase of the test, that can be defined as the phase of isothermal crystallisation of the material, the rheometers record the increase, as a function of time, of the Elastic Modulus G', both for the sample that has been subjected to a pre-application of the above-mentioned shear-stress, and for the similar sample that has received no shear-stress.

The duration of this crystallisation step can be variable, depending on the characteristic of the material, from a few minutes to several hours.

This measurement is considered as finished when the Elastic Modulus of the tested sample does not grow anymore with time and reaches a final and constant asymptotic value, that is typical of that material itself.

As said, all the materials under test are subjected to the same crystallization test, after a step in the molten state at 140°C, both in the absence and in the presence of a pre-imposed shear-stress for 600 seconds.

In the first case, the rheometer records a crystallisation curve of the material (expressed as the increase in time of the Elastic Modulus G') without the possible effect of a pre-imposed shear-stress, that might in case modify its crystallisation kinetics. On the contrary, in the second case, the rheometer records the analogous curve of the increase in time of the Elastic Modulus G', for checking whether or not the pre-imposed shear-stress (at the shear-rate of 50 s⁻¹, for 600 seconds at 140°C) is causing a significant anticipation and acceleration of the material crystallisation; in this way showing that that polymer or blend of polymers will certainly generate a severe problem of "die build-up" when extruded by slot-die coating.

Therefore, polymeric materials whose crystallisation kinetics is significantly influenced by the presence or absence of an applied shear-stress, will exhibit a curve of the increase of G' as a function of time, that is different, depending on the fact that a shear-stress has been or not applied on the material.

In particular, when such pre-applied shear-stress is null, the starting in the increase of the value of G' will occur at a time, after the starting of the test, that is longer; on the contrary when a pre-applied shear-stress anticipates and accelerates the kinetics of the crystallisation, said starting of the increase in the value of G' will occur at a shorter time.

If we refer to a time t(no-shear)₀ (time at which the crystallisation starts and the Elastic Modulus begins to increase, in a material that has not been subjected to any shear-stress) and to a time t(shear)₀ (time at which the crystallisation starts and the Elastic Modulus begins to increase, in a material that on the contrary has received a pre-applied shear-stress), we will have t(shear)_{0<} t(no-shear)₀ for all those polymeric materials whose kinetics of crystallisation is influenced by an applied shear-stress; while we will have that t(shear)₀₌t(no-shear)₀ for all those polymeric materials whose kinetics of crystallisation is not affected by the presence of an applied shear stress. In this latest case, the two curves of the increase of G' as a function of time, in the two parallel experiment, with and without a pre-applied shear-stress, will substantially coincide (apart from the possible presence of experimental fluctuations).

Therefore, the polymers and the polymeric blends that undergo an anticipation and an acceleration of their crystallisation kinetics after a pre-application of a shear-stress (and that therefore will cause die build-up in slot-die coating) show a starting point of the increase of G' at a time that is significantly shorter and is anticipated compared to the similar curve that the same material shows when it has not been previously subjected to a shear-stress.

It is important to notice that in both tests, with and without the pre-applied shear stress, the final asymptotic value reached by G' is exactly the same, because this value depends only on the intrinsic capability by a certain polymer or by a certain polymeric blend of developing a certain level of final crystallinity (i.e. a certain final value of G') that is independent of the kinetics by which this level of final crystallinity is reached.

The effect of the shear-stress on the material's crystallinity is consequently just a kinetic effect, an effect of anticipation and acceleration of said crystallisation and it is not an effect that may influence the final quantity of formed crystals.

On the contrary, polymers or polymeric blends whose kinetics of crystallisation is not sensitive or is scarcely sensitive to applied shear-stresses (and that therefore will not show die build-up when processed by slot-die coating) will show substantially the same curve of the increase of Elastic Modulus G' versus time, in both tests, the one with the application of the shear-stress and the one without any shear-stress.

Practically, for comparing and measuring in a quantitative way how much the two curves of G' as a function of time are similar or different, one can act in the following way: after having identified the two times t(no-shear)₀ and t(shear) ₀ at which the two curves of the increase of G' as a function of time start to increase over the initial value, one calculates, at the same time and at subsequent intervals of 10 seconds, for each value of time, the ratio G' (t shear) / G' (t no-shear), where G' (t shear) is the value at a generic time of the Elastic Modulus G' in the curve with a pre-application of a shear-stress; while G' (t no-shear) is the value of G' at the same generic time in the curve without any application of a shear-stress. This calculation is done in consecutive points, that at 10 seconds one after the other, until both the two curves return to coincide at the final asymptotic value of G'.

If at each measured value of time, G' (t shear) is greater than G' (t no-shear) for no more than 100%, or also if the ratio G' (t shear) / G' (t no-shear) never exceeds the maximum value of 2.0, the two curves, with and without the pre-application of a fixed shear-stress, are considered to be substantially coincident one with the other; and the tested polymer or blend of compatible polymers are defined as having a kinetics of crystallisation that is not affected by an applied shear-stress, according to the herein described test. Said polymer or blend of compatible polymers is hence suitable for being used as the basic polymeric component in the hot-melt adhesive formulations according to the present invention.

### Quantitative measurement of crystallinity in polymeric materials by Differential Scanning Calorimetry (DSC)

All the crystallinities of all the herein described polymeric materials are detected and measured in DSC (Differential Scanning Calorimetry) graph, through the presence of melting temperature peaks (or of crystallisation peaks) of the crystalline fractions in the polymeric materials, according to the following procedures.

All the DSC experiments, both in crystallisation cycles, in decreasing temperature, or in melting cycles, in increasing temperature, are performed following the ASTM Method D 3417-99.

Said cycles are performed between the temperatures of - 70°C and 180°C (and vice versa) for having complete information on all the phenomena occurring inside the material, for example for measuring its Glass Transition Temperature, Tg.

The Melting and Crystallisation Enthalpies are expressed in J/g and are given by the area of the peak, calculated by integration, or by the sum of all the possible peaks that appear during a certain DSC cycle. The DSC experiments on the polymers or on the blends of polymers comprised in the hot-melt adhesive formulations of the present invention are performed at a range temperature (both in increase and in decrease) equal to 10°C/minute, as recommended by ASTM D 3417-99.

### Qualitative measurement of the "Die Drool" / "Die Build-Up" phenomenon

As already mentioned, the "die drool" or "die build-up" phenomenon, i.e. a build-up of extruded material on the extrusion-head, must be avoided by all means, as taught by the present invention, because it has highly detrimental consequences on the application process of the adhesives and on the quality of the manufactured articles, like gross inhomogeneity in the coated adhesive layer; absence of adhesive in certain areas and presence of big lumps in other areas; fouling of the lines etc.

The behaviour as "die build-up" for the present adhesives is herein qualitatively checked under typical conditions that mimic the operating conditions of industrial lines in the manufacturing of absorbent hygienic articles.

In particular, the presence or absence of the "die drool phenomenon" is checked by extruding the tested hot-melt adhesive in slot-die coating, at high line speed, on a polypropylene nonwoven, with the basis weight of 12 g/m², supplied by Union Industries (Italy).

On a pilot-line mimicking the working conditions of an industrial line for hygienic articles and operating at the speed of at least 250 m/minute, the tested adhesive is extruded and coated on the nonwoven, at a basis weight of 3 g/m² and at the temperature of at least 150°C. The presence or absence of a visible die build-up is visually observed through the easily visible accumulation of a layer of material that remains adhered on metal of the extrusion-head. Generally, this adhered layer has a thickness of at least from about 2 to about 5 mm (or even more in some cases) and it is in length / width as wide as the extruding hole. Said layer of material that accumulates on the head and that continues to grow with time, occasionally and irregularly breaks and detaches, forming massive lumps that are dragged away by the running nonwoven while the build-up restarts.

In the conditions of flow for the adhesive as specified above, when a significant die build-up occurs, it is possible to notice, already after about 10 to 15 seconds from the starting of the experiment, and in all cases within 60 seconds of run, a well visible accumulation of material on the extrusion-head.

If, on the contrary, no visible accumulation of the extruded adhesive is observed after at least 120 seconds from the starting of the experiment, one can conclude that the tested material does not exhibit any phenomenon of die drool / die build-up.

### EXAMPLES

The polymers (A-B-C) and the blends of polymers (D-E-F, with composition expressed in percent by weight) that have been tested with the above described test of Induced Crystallisation under shear-stress before being formulated, together with all the other ingredients, inside the hot-melt adhesive formulations shown in the following Examples are shown in Table 1.

For each polymer or blend of polymers, Table 1 shows:
- the Polydispersity Index Mw / Mn of the polymer or blend of polymers;
- the temperature at which the crystallisation test is performed;
- the value in seconds of t(no-shear)_{asympt}, i.e. the time at which the curve of G' as a function of time, in the experiment without the pre-application of any shear-stress, reaches its final asymptotic value;
- the maximum value of the ratio G' (t shear) / G' (t no-shear) between the starting of the crystallisation and its completion at the final asymptotic value of G', where the two curves, with and without the pre-application of a shear-stress, coincide again.

As already described, polymers and blends of polymers that show a maximum value of said ratio G' (t shear) / G' (t no-shear) that is not greater than 2.0, are considered to pass the test because, when they are formulated into hot-melt adhesive formulations, such formulations do not show any significant detrimental phenomenon of die-drool / die build-up when processed, even on high speed lines, by slot-die coating and in general by extrusion.

**TABLE 1**

| | A | B | C | D | E | F |
|---|---|---|---|---|---|---|
| Koattro PB M 1500M | 100% | | | 90% | 90% | 60% |
| Koattro PB M 8510M | | 100% | | 10% | | 10% |
| Koattro PB 0801M | | | 100% | | 10% | 30% |
| | | | | | | |
| Polydispersity Index | 2.26 | 2.05 | 4.88 | 2.46 | 2.50 | 3.17 |
| T test (°C) | 70 | 85 | 95 | 70 | 70 | 90 |
| t (no-shear)_{asympt}, (s) | 5, 460 | 41220 | 374 | 550 | 629 | 662 |
| Maximum value of ratio G' (t shear) / G' (t no-shear) | 4,2 | 2.21 | 4,3 | 0,76 | 0,57 | 1,05 |
| Expected Die Build-up | YES | YES | YES | NO | NO | NO |

### EXAMPLES ACCORDING TO THE INVENTION

The present invention is better illustrated by the following examples, which are given herein merely for the purpose of illustration and are not to be regarded as limiting the scope of the invention or the manner in which it can be practiced. Unless specifically indicated otherwise, parts and percentages are given by weight.

### EXAMPLE 1

| The following hot-melt adhesive formulation according to the present invention has been prepared by mixing its components in the molten state at 170°C.Ingredient | % by weight on the total weight of the hot-melt adhesive formulation | Nature and Supplier |
|---|---|---|
| Koattro PB M 1500M | 44.1 | Metallocenic bimodal polymer composition of isotactic butene-1, supplied by LyondellBasell |
| Koattro PB M 8510M | 4.9 | Metallocenic bimodal polymer composition of isotactic butene-1, supplied by LyondellBasell |
| Regalite R 1100 | 44.2 | Fully hydrogenated hydrocarbon tackifier supplied by Eastman |
| Polywax 850 | 1.0 | Polyethylene homopolymer wax supplied by Baker Hughes |
| Primol 352 | 3.8 | Viscosity modifier (paraffinic mineral oil) supplied by ExxonMobil |
| Irganox 1010 | 2.0 | Antioxidant supplied by BASF |

The hot-melt adhesive formulation of Example 1 comprises the polymeric blend D of Table 1; said blend has a Polydispersity Index Mw/Mn equal to 2.46.

Said polymeric blend, in the test of Induced Crystallisation under Shear-Stress has shown a very small value of the ratio G' (t shear) / G' (t no-shear) that in fact in said test has reached a maximum value as low as 0.76. In fact, when the adhesive formulation of Example 1 has been extruded by slot-die coating, at the temperature of 160°C and at the very high line speed of 250 m/minute, it has not showed any die build-up, even after more than 30 minutes of continuous run.
The above adhesive formulation has a viscosity at 170°C, measured according to the Method ASTM D 3236 - 88, of 2,700 mPa.s; a R&B Softening Point of 88.1°C; an Elastic Modulus G' at 23°C and 1 Hz equal to 1.51 MPa and a G' at 37°C and 1 Hz equal to 0.69 MPa. Moreover, the present adhesive formulation has a fusion Enthalpy, after five days of aging at room conditions, of 15.2 J/g.

Again after five days of aging under room conditions, its mechanical properties, measured at 23°C and at the frequency of 0.01 Hz in an experiment of Stress vs. Elongation at Break, according to the method described above, are the following ones: the Elongation at Breaks exceeds the maximum value that can be read in the test, i.e. it is greater than 600%. At such maximum readable value of Elongation, the maximum value of Stress (peak) of Example 1 is equal to 3.8 MPa and the correspondent Toughness is equal to 3.6 MJ/m³.

### EXAMPLE 2

The following hot-melt adhesive formulation according to the present invention has been prepared by mixing its components in the molten state at 170°C.

| Ingredient | % by weight on the total weight of the hot-melt adhesive formulation | Nature and Supplier |
|---|---|---|
| Koattro PB M 1500M | 44.1 | Metallocenic bimodal polymer composition of isotactic butene-1, supplied by LyondellBasell |
| Koattro PB 0801M | 4.9 | Isotactic polybutene-1, supplied by LyondellBasell |
| Regalite R 1100 | 44.2 | Fully hydrogenated hydrocarbon tackifier supplied by Eastman |
| Polywax 850 | 1.0 | Polyethylene homopolymer wax supplied by Baker Hughes |
| Primol 352 | 3.8 | Viscosity modifier (paraffinic mineral oil) supplied by ExxonMobil |
| Irganox 1010 | 2.0 | Antioxidant supplied by BASF |

The hot-melt adhesive formulation of Example 2 comprises the polymeric blend E of Table 1; said blend has a Polydispersity Index Mw/Mn equal to 2.50.

In the test of Induced Crystallisation under Shear-Stress, the polymeric blend comprised in Example 2 has shown a maximum value of the ratio G' (t shear) / G' (t no-shear) that is just 0.57. In fact, when the adhesive formulation of Example 2 has been extruded by slot-die coating, at the temperature of 160°C and even at the very high line speed of 250 m/minute, it has not showed any die build-up even after times longer than 30 minutes of continuous run.
The above adhesive formulation has a viscosity at 170°C, measured according to the Method ASTM D 3236 - 88, of 2,190 mPa.s; a R&B Softening Point of 91.1°C; an Elastic Modulus G' at 23°C and 1 Hz equal to 1.66 MPa and a G' at 37°C and 1 Hz equal to 0.81 MPa. The adhesive formulation if Example 2 has also a fusion Enthalpy, after five days of aging at room conditions, of 16.1 J/g.

The mechanical properties, at 23°C and at the frequency of 0.01 Hz in an experiment of Stress vs. Elongation at Break, according to the method described above, of the adhesive formulation of Example 2, after aging for five days at room conditions, are the following ones: again, the Elongation at Breaks exceeds the maximum value readable in the test, i.e. it is greater than 600%. At such maximum readable value of Elongation, the maximum value of Stress (peak) of Example 2 is equal to 4.1 MPa and the correspondent Toughness is equal to 3.8 MJ/m³.

### EXAMPLE 3

The following hot-melt adhesive formulation according to the present invention has been prepared by mixing its components in the molten state at 170°C.

| Ingredient | % by weight on the total weight of the hot-melt adhesive formulation | Nature and Supplier |
|---|---|---|
| Koattro PB M 1500M | 29.4 | Metallocenic bimodal polymer composition of isotactic butene-1, supplied by LyondellBasell |
| Koattro PB M 8510M | 4.9 | Metallocenic bimodal polymer composition of isotactic butene-1, supplied by LyondellBasell |
| Koattro PB 0801M | 14.7 | Isotactic polybutene-1, supplied by LyondellBasell |
| Regalite R 1100 | 44.2 | Fully hydrogenated hydrocarbon tackifier supplied by Eastman |
| Polywax 850 | 1.0 | Polyethylene homopolymer wax supplied by Baker Hughes |
| Primol 352 | 3.8 | Viscosity modifier (paraffinic mineral oil) supplied by ExxonMobil |
| Irganox 1010 | 2.0 | Antioxidant supplied by BASF |

The hot-melt adhesive formulation of Example 3 comprises the polymeric blend F of Table 1; said blend has a Polydispersity Index Mw/Mn equal to 3.17.

In the test of Induced Crystallisation under Shear-Stress, the polymeric blend comprised in Example 2 has shown a maximum value of the ratio G' (t shear) / G' (t no-shear) that is just 1.05. In fact, when the adhesive formulation of Example 3 has been extruded by slot-die coating, at the temperature of 160°C and even at the very high line speed of 250 m/minute, it has not showed any die build-up, even after times longer than 30 minutes of continuous run.
The above adhesive formulation has a viscosity at 170°C, measured according to the Method ASTM D 3236 - 88, of 4,380 mPa.s; a R&B Softening Point of 99.8°C; an Elastic Modulus G' at 23°C and 1 Hz equal to 1.81 MPa and a G' at 37°C and 1 Hz equal to 0.85 MPa. The adhesive formulation if Example 2 has also a fusion Enthalpy, after five days of aging under room conditions, of 21.3 J/g.

The mechanical properties, at 23°C and at the frequency of 0.01 Hz in an experiment of Stress vs. Elongation at Break, according to the method described above, after aging for five days under room conditions, of the adhesive formulation of Example 3 are the following ones: once again the Elongation at Breaks exceeds the maximum value readable in the test, i.e. it is greater than 600%. At such maximum readable value of Elongation, the maximum value of Stress (peak) of Example 3 is equal to 11.3 MPa and the correspondent Toughness is equal to 8.1 MJ/m³.

### COMPARATIVE EXAMPLE 1

The following Comparative Example 1 has been prepared by mixing its components in the molten state at 170°C.

| Ingredient | % by weight on the total weight of the hot-melt adhesive formulation | Nature and Supplier |
|---|---|---|
| Koattro PB M 1500M | 49.0 | Metallocenic bimodal polymer composition of isotactic butene-1, supplied by LyondellBasell |
| Regalite R 1100 | 44.2 | Fully hydrogenated hydrocarbon tackifier supplied by Eastman |
| Polywax 850 | 1.0 | Polyethylene homopolymer wax supplied by Baker Hughes |
| Primol 352 | 3.8 | Viscosity modifier (paraffinic mineral oil) supplied by ExxonMobil |
| Irganox 1010 | 2.0 | Antioxidant supplied by BASF |

The hot-melt adhesive formulation of Comparative Example 1 comprises, as its basic polymeric component, Koattro PB M 1500M that has a Polydispersity Index Mw/Mn equal to 2.26.

In the test of Induced Crystallisation under Shear-Stress, Koattro PB M 1500M has shown a maximum value of the ratio G' (t shear) / G' (t no-shear) as high as 4.2, in this way indicating that its crystallisation kinetics is very strongly influenced by the application of a shear-stress. In fact, when the adhesive formulation of Comparative Example 1 has been extruded by slot-die coating, at the temperature of 160°C and at the line speed of 250 m/minute, already after just five seconds of run it has shown a very strong and unacceptable build-up of material on the extrusion head.
The above adhesive comparative formulation has a viscosity at 170°C, measured according to the Method ASTM D 3236 - 88, of 1,900 mPa.s; a R&B Softening Point of 88.9°C; an Elastic Modulus G' at 23°C and 1 Hz equal to 1.19 MPa and a G' at 37°C and 1 Hz equal to 0.54 MPa. The adhesive formulation if Example 2 has also a fusion Enthalpy, after five days of aging under room conditions, of 13.6 J/g.

The mechanical properties, at 23°C and at the frequency of 0.01 Hz in an experiment of Stress vs. Elongation at Break, according to the method described above, after aging for five days under room conditions, of the adhesive formulation of Comparative Example 1 are as follows: also in this case the Elongation at Break exceeds the maximum value readable in the test, i.e. it is greater than 600%. At such maximum readable value of Elongation, the maximum value of Stress (peak) of Comparative Example 1 is equal to 3.4 MPa and the correspondent Toughness is equal to 3.3 MJ/m³.

## Claims

1. Hot-melt adhesive formulation, which comprises at least a polymer, that can be a homopolymer or a copolymer, or a compatible blend of polymers, that can be homopolymers or copolymers, wherein said polymer or compatible blend of polymers comprises a homopolymer of olefins from C2 to C12 or a copolymer among the same olefins, **characterised in that** said polymer or compatible blend of polymers:
A) has a crystallisation kinetics that is not affected by the pre-application of a shear-stress, according to the test of "Induced Crystallisation under shear-stress", as herein described;
B) has an Index of Polydispersity that is not lower than 2.0 and preferably not lower than 2.5;
said hot-melt adhesive formulation being further **characterised in that** it has:
- a Brookfield viscosity at 170°C, measured according to the Method ASTM D 3236 - 88, ranging between 500 mPa.s and 50,000 mPa.s
- a Ring & Ball Softening Temperature, measured according to the Method ASTM D 36 - 95, not higher than 150°C
- an Elastic Modulus G', measured at 23°C and at a frequency of 1 Hz, that is not lower than 0.05 MPa.

2. Hot-melt adhesive formulation as in 1), **characterised in that** the polymer or the compatible blend of polymers, when tested in a DSC melting test, after 24 hours of aging under room conditions, shows a peak whose final highest temperature of end of the melting, is not lower than 70°C.

3. Hot-melt adhesive formulation according to any of the preceding claims from 1) and 2), that, when tested in a DSC melting test, after aging for five days at room conditions, shows a melting Enthalpy that is not greater than 40 J/g.

4. Hot-melt adhesive formulation according to any of the preceding claims from 1) to 3), in which the polymer or the compatible blend of polymers constitutes from 10% by weight to 99.5% by weight of said adhesive hot-melt adhesive formulation, and preferably from 20% by weight to 90% by weight.

5. Hot-melt adhesive formulation according to any of the preceding claims from 1) to 4), that comprises at least one tackifier having a Ring & Ball Softening Point, measured according to the Method ASTM D 36 - 95, ranging from 5°C to 160°C.

6. Hot-melt adhesive formulation as in 5), **characterised in that** the tackifier or the blend of tackifiers are selected from aliphatic hydrocarbon tackifiers and their fully or partially hydrogenated derivatives; aromatic hydrocarbon tackifiers and their fully or partially hydrogenated derivatives; aliphatic/aromatic hydrocarbon tackifiers and their fully or partially hydrogenated derivatives; terpene tackifiers and modified terpene tackifiers and their fully or partially hydrogenated derivatives; rosins, their esters and their fully or partially hydrogenated derivatives, and their blends.

7. Hot-melt adhesive formulation as in 5) or 6), **characterised in that** the tackifier or the blend of tackifiers have a Ring & Ball Softening Point, measured according to the Method ASTM D 36 - 95, between 70°C and 135°C, preferably between 80°C and 125°C.

8. Hot-melt adhesive formulation according to any of the preceding claims from 5) to 7), **characterised in that** the tackifier or the blend of tackifiers have a content of volatile impurities that is not greater than 5 ppm and preferably not greater than 1 ppm.

9. Hot-melt adhesive formulation according to any of the preceding claims from 5) to 8), **characterised in that** the tackifier or the blend of tackifiers constitutes from zero to 75% by weight of the adhesive formulation, preferably from 10% by weight to 70% by weight and more preferably from 20% by weight to 65% by weight.

10. Hot-melt adhesive formulation according to any of the preceding claims from 1) to 9), that comprises at least one plasticiser, that is liquid at room temperature, or a blend of plasticisers, that is liquid at room temperature.

11. Hot-melt adhesive formulation as in 10), **characterised in that** the plasticiser, that is liquid at room temperature, or the blend of plasticisers, that is liquid at room temperature, are selected from paraffinic mineral oils; naphthenic mineral oils; paraffinic and naphthenic hydrocarbons which are liquid at room temperature, and blends thereof; oligomers which are liquid at room temperature of polyolefins and their copolymers, such as liquid oligomers derived from ethylene, propylene, butene, iso-butylene, copolymers thereof and the like; plasticisers which are liquid at room temperature, formed by esters, such as phthalates, benzoates, sebacates; vegetable oils; natural and synthetic fats; and blends thereof;

12. Hot-melt adhesive formulation as in 10) and in 11), **characterised in that** the plasticisers, that is liquid at room temperature, or the blend of plasticisers, that is liquid at room temperature, is formed by a oligomer or by a blend of oligomers of synthetic poly-alpha-olefins, synthesized from olefins from C2 to C20 and with an Average Number Molecular Weight comprised between 150 g/mol and 15,000 g/mol.

13. Hot-melt adhesive formulation according to any of the preceding claims from 10) to 12), **characterised in that** the plasticisers, that is liquid at room temperature, or the blend of plasticisers, that is liquid at room temperature, constitutes from zero to 50% by weight of the adhesive formulation, preferably from 5% by weight to 40% by weight and more preferably from 10% by weight to 35% by weight.

14. Hot-melt adhesive formulation according to any of the preceding claims from 1) to 13), **characterised in that** the plasticiser, that is liquid at room temperature, or the blend of plasticisers, comprises at least one wax or a blend of waxes, having a dropping-point, measured according to ASTM D-3954-94, comprised between 40°C and 170°C.

15. Hot-melt adhesive formulation as in 14), **characterised in that** the wax or the blend of waxes is selected from paraffinic waxes and especially waxes synthesised from olefins from C2 to C10, and their blends; hydrocarbon waxes from C12 to C40, also in their derivatives modified with acidic carboxyl groups or alcoholic groups; copolymer waxes synthesised from ethylene and maleic anhydride or from propylene and maleic anhydride; microcrystalline waxes; waxes formed by esters of fatty acids from C12 to C40; natural waxes like beeswax, carnauba wax, montan wax and similar; and their blends.

16. Hot-melt adhesive formulation as in 14) and in 15), **characterised in that** the wax or the blend of waxes constitutes from zero to 20% by weight of the adhesive formulation, preferably from zero to 10% by weight, and more preferably from zero to 5% by weight.

17. Hot-melt adhesive formulation according to any of the preceding claims from 1) to 16), that, after aging for five days under room conditions, has a curve Stress / Elongation at Break, measured at 23°C and at the frequency of 0.01 Hz according to the method described herein, which shows a maximum stress (peak) not lower than 0.15 MPa.

18. Hot-melt adhesive formulation according to any of the preceding claims from 1) to 16), that, after aging for five days under room conditions, has a curve Stress/ Elongation at Break, measured at 23°C and at the frequency of 0.01 Hz according to the method described herein, which shows elongation at break not lower than 50%.

19. Hot-melt adhesive formulation according to any of the preceding claims from 1) to 16), that, after aging for five days under room conditions, has a curve Stress / Elongation at Break, measured at 23°C and at the frequency of 0.01 Hz according to the method described herein, which shows a toughness not lower than 0.2 MJ/m³.

20. Hot-melt adhesive formulation according to any of the preceding claims from 1) to 19), that has an Elastic Modulus G', measured at 23°C and at the frequency of 1 Hz according to the method described herein, that is not lower than 0.5 MPa.

21. Hot-melt adhesive formulation according to any of the preceding claims from 1) to 19), that has an Elastic Modulus G', measured at 37°C and at the frequency of 1 Hz according to the method described herein, that is not lower than 0.1 MPa.

22. A bonded structure comprising:
- a first substrate
- a second substrate
- a hot-melt adhesive formulation according to any one of the preceding claims from 1) to 21), bonding the first and second substrate, which, when applied at a basis weight between 0.5 g/m² and 50 g/m², gives to the bonded structure a Peel Strength greater than 0.25 N per 50 mm width.

23. A hygienic absorbent article, comprising the hot-melt adhesive formulation according to any one of the preceding claims 1) to 21).

24. An absorbent hygienic article, comprising a bonded structure as in 22).

25. An article as in 23) or 24), **characterised in that** said article is a baby-diaper, a training pants diaper, a diaper for incontinent adults, a feminine catamenial pad;

26. An absorbent hygienic article according to any one of the preceding claims from 23) to 25), **characterised in that** the hot-melt adhesive formulation is used for at least one of the following uses: i) general construction adhesive of the whole article; ii) for bonding elastic components (threads, ribbons, films or elastic panels, etc.); iii) for strengthening and ensuring, even in use, the integrity of the absorbent core of the absorbent hygienic article; iv) for the bonding of perforated films both with a bidimensional or tridimensional structure; v) for the bonding of a nonwoven with another nonwoven or with a plastic film.

27. An article comprising the hot-melt adhesive formulation according to any one of the preceding claims 1) to 21), **characterised in that** said article is an absorbent surgical mattress or sheet or surgery laminate for medical use or a wound-dressing product.

28. An article comprising the hot-melt adhesive formulation according to any one of the preceding claims 1) to 21), **characterised in that** said article is a mattress or a component thereof.

29. An article comprising the hot-melt adhesive formulation according to any one of the preceding claims 1) to 21), **characterised in that** said article is a packaging.

30. An article according to any one of the preceding claims 23) to 29), **characterised in that** the hot-melt adhesive formulation is applied in at least one use by the process of slot-die coating.

## Patentansprüche

1. Schmelzklebstoffzubereitung, die wenigstens ein Polymer umfasst, das ein Homopolymer oder ein Copolymer sein kann, oder eine kompatible Mischung von Polymeren, die Homopolymere oder Copolymere sein können, wobei besagte(s) Polymer oder kompatible Mischung von Polymeren ein Homopolymer von Olefinen von C2 bis C12 oder ein Copolymer unter den gleichen Olefinen umfasst, **dadurch gekennzeichnet, dass** besagte(s) Polymer oder kompatible Mischung von Polymeren:
A) eine Kristallisationskinetik aufweist, die nicht durch die Vor-Beaufschlagung einer Schubspannung beinträchtig wird, gemäß dem Test von "Induzierte Kristallisation unter Schubspannung", wie hierin beschrieben;
B) einen Polydispersitätsindex ausweist, der nicht kleiner als 2,0 und bevorzugt nicht kleiner als 2,5 ist;
wobei die Schmelzklebstoffzubereitung ferner **dadurch gekennzeichnet ist, dass** sie:
- eine Brookfield-Viskosität bei 170°C, gemessen gemäß dem Verfahren ASTM D 3236-88, im Bereich zwischen 500 mPa·s bis 50.000 mPa·s aufweist,
- eine Ring- & Ball-Erweichungstemperatur, gemessen gemäß dem Verfahren ASTM D 36-95, von nicht höher als 150°C aufweist,
- einen Elastizitätsmodul G', gemessen bei 23°C und bei einer Frequenz von 1 Hz, aufweist, der nicht kleiner als 0,05 MPa ist.

2. Schmelzklebstoffzubereitung nach Anspruch 1, **dadurch gekennzeichnet, dass** das Polymer oder die kompatible Mischung von Polymeren, bei Testung in einem DSC-Schmelztest, nach 24 Stunden Alterung unter Raumbedingungen, einen Peak zeigt, dessen endgültige höchste Temperatur beim Ende des Schmelzens nicht kleiner als 70°C ist.

3. Schmelzklebstoffzubereitung nach einem der vorangehenden Ansprüche 1 und 2, die, bei Testung in einem DSC-Schmelztest, nach Alterung für fünf Tage bei Raumbedingungen, eine Schmelzenthalpie zeigt, die nicht größer als 40 J/g ist.

4. Schmelzklebstoffzubereitung nach einem der vorangehenden Ansprüche 1 bis 3, bei der das Polymer oder die kompatible Mischung von Polymeren 10 Gew.-% bis 99,5 Gew.- % der Schmelzklebstoffzubereitung ausmacht, und bevorzugt 20 Gew.-% bis 90 Gew.-%.

5. Schmelzklebstoffzubereitung nach einem der vorangehenden Ansprüche 1 bis 4, die wenigstens einen Klebrigmacher mit einem Ring-& Ball-Erweichungspunkt, gemessen gemäß dem Verfahren ASTM D 36-95, in einem Bereich von 5°C bis 160°C umfasst.

6. Schmelzklebstoffzubereitung nach Anspruch 5, **dadurch gekennzeichnet, dass** der Klebrigmacher oder die Mischung von Klebrigmachern ausgewählt ist aus aliphatischen Kohlenwasserstoff-Klebrigmachern und deren vollständig oder teilweise hydrierten Derivaten; aromatischen Kohlenwasserstoff-Klebrigmachern und deren vollständig oder teilweise hydrierten Derivaten; aliphatischen/aromatischen Kohlenwasserstoff-Klebrigmachern und deren vollständig oder teilweise hydrierten Derivaten; Terpen-Klebrigmachern und modifizierten Terpen-Klebrigmachern und deren vollständig oder teilweise hydrierten Derivaten; Kolophoniumharzen, deren Estern und deren vollständig oder teilweise hydrierten Derivaten sowie deren Mischungen.

7. Schmelzklebstoffzubereitung nach Anspruch 5 oder 6, **dadurch gekennzeichnet, dass** der Klebrigmacher oder die Mischung von Klebrigmachern einen Ring- & Ball-Erweichungspunkt, gemessen gemäß dem Verfahren ASTM D 36-95, zwischen 70°C und 135°C aufweist, bevorzugt zwischen 80°C und 125°C.

8. Schmelzklebstoffzubereitung nach einem der vorangehenden Ansprüche 5 bis 7, **dadurch gekennzeichnet, dass** der Klebrigmacher oder die Mischung von Klebrigmachern einen Gehalt an flüchtigen Verunreinigungen aufweist, der nicht größer als 5 ppm und bevorzugt nicht größer als 1 ppm ist.

9. Schmelzklebstoffzubereitung nach einem der vorangehenden Ansprüche 5 bis 8, **dadurch gekennzeichnet, dass** der Klebrigmacher oder die Mischung von Klebrigmachern 0 bis 75 Gew.-% der Klebstoffzubereitung ausmacht, vorzugsweise 10 Gew.-% bis 70 Gew.-% und noch bevorzugter 20 Gew.-% bis 65 Gew.-% ausmacht.

10. Schmelzklebstoffzubereitung nach einem der vorangehenden Ansprüche 1 bis 9), die wenigstens einen Weichmacher umfasst, der bei Raumtemperatur flüssig ist, oder eine Mischung von Weichmachern, die bei Raumtemperatur flüssig ist.

11. Schmelzklebstoffzubereitung nach Anspruch 10, **dadurch gekennzeichnet, dass** der Weichmacher, der bei Raumtemperatur flüssig ist, oder die Mischung von Weichmachern, die bei Raumtemperatur flüssig ist, ausgewählt ist aus paraffinischen Mineralölen; naphthenischen Mineralölen; paraffinischen und naphthenischen Kohlenwasserstoffen, die bei Raumtemperatur flüssig sind, und Mischungen derselben; Oligomeren, die bei Raumtemperatur flüssig sind, von Polyolefinen und deren Copolymeren, wie flüssigen Oligomeren, abgeleitet von Ethylen, Propylen, Buten, Isobutylen, Copolymeren derselben und dergleichen; Weichmachern, die bei Raumtemperatur flüssig sind, gebildet aus Estern wie Phthalaten, Benzoaten, Sebacaten; pflanzlichen Ölen; natürlichen und synthetischen Fetten; und Mischungen derselben.

12. Schmelzklebstoffzubereitung nach Anspruch 10 und Anspruch 11, **dadurch gekennzeichnet, dass** die Weichmacher, die bei Raumtemperatur flüssig sind, oder die Mischung von Weichmachern, die bei Raumtemperatur flüssig ist, gebildet wird durch ein Oligomer oder durch eine Mischung von Oligomeren von synthetischen Polyalphaolefinen, synthetisiert aus Olefinen von C2 bis C20 und mit einem Zahlenmittelmolgewicht, das zwischen 150 g/mol und 15.000 g/mol umfasst ist.

13. Schmelzklebstoffzubereitung nach einem der vorangehenden Ansprüche 10 bis 12, **dadurch gekennzeichnet, dass** der Weichmacher, der bei Raumtemperatur flüssig ist, oder die Mischung von Weichmachern, die bei Raumtemperatur flüssig ist, 0 bis 50 Gew.-% der Klebstoffzubereitung, bevorzugt 5 Gew.-% bis 40 Gew.-% und noch bevorzugter 10 Gew.-% bis 35 Gew.-%, ausmacht.

14. Schmelzklebstoffzubereitung nach einem der vorangehenden Ansprüche 1 bis 13, **dadurch gekennzeichnet, dass** der Weichmacher, der bei Raumtemperatur flüssig ist, oder die Mischung von Weichmachern, wenigstens ein Wachs oder eine Mischung von Wachsen, mit einem Tropfpunkt, gemessen gemäß ASTM D-3954-94, zwischen 40°C und 170°C umfasst.

15. Schmelzklebstoffzubereitung nach Anspruch 14, **dadurch gekennzeichnet, dass** das Wachs oder die Mischung von Wachsen ausgewählt ist aus paraffinischen Wachsen und insbesondere Wachsen, synthetisiert aus Olefinen von C2 bis C10, und deren Mischungen; Kohlenwasserstoffwachsen von C12 bis C40, ebenfalls in ihren Derivaten modifiziert mit sauren Carboxylgruppen oder alkoholischen Gruppen; Copolymerwachsen, synthetisiert aus Ethylen und Maleinsäureanhydrid oder aus Propylen und Maleinsäureanhydrid; mikrokristallinen Wachsen; Wachsen aus Estern von Fettsäuren aus C12 bis C40; natürlichen Wachsen wie Bienenwachs, Carnaubawachs, Montanwachs und ähnlichen; und deren Mischungen.

16. Schmelzklebstoffzubereitung nach Anspruch 14 und Anspruch 15, **dadurch gekennzeichnet, dass** das Wachs oder die Mischung von Wachsen 0 bis 20 Gew.-% der Klebstoffzubereitung, bevorzugt 0 bis 10 Gew.-% und noch bevorzugter 0 bis 5 Gew.-% ausmacht.

17. Schmelzklebstoffzubereitung nach einem der vorangehenden Ansprüche 1 bis 16, die, nach Alterung für fünf Tage unter Raumbedingungen, eine Spannungs-Bruchdehnungs-Kurve, gemessen bei 23°C und bei der Frequenz von 0,01 Hz gemäß dem hierin beschriebenen Verfahren, aufweist, die eine maximale Spannung (peak) von nicht kleiner als 0,15 MPa zeigt.

18. Schmelzklebstoffzubereitung nach einem der vorangehenden Ansprüche 1 bis 16, die, nach Alterung für fünf Tage unter Raumbedingungen, eine Spannungs-Bruchdehnungs-Kurve, gemessen bei 23°C und bei der Frequenz von 0,01 Hz gemäß dem hierin beschriebenen Verfahren, aufweist, die eine Bruchdehnung von nicht kleiner als 50 % zeigt.

19. Schmelzklebstoffzubereitung nach einem der vorangehenden Ansprüche 1 bis 16, die, nach Alterung für fünf Tage unter Raumbedingungen, eine Spannungs-Bruchdehnungs-Kurve, gemessen bei 23°C und bei der Frequenz von 0,01 Hz gemäß dem hier beschriebenen Verfahren, aufweist, die eine Zähigkeit von nicht kleiner als 0,2 MJ/m³ zeigt.

20. Schmelzklebstoffzubereitung nach einem der vorangehenden Ansprüche 1 bis 19, die einen Elastizitätsmodul G', gemessen bei 23°C und bei der Frequenz von 1 Hz gemäß dem hierin beschriebenen Verfahren, aufweist, der nicht kleiner als 0,5 MPa ist.

21. Schmelzklebstoffzubereitung nach einem der vorangehenden Ansprüche 1 bis 19, die einen Elastizitätsmodul G', gemessen bei 37°C und bei der Frequenz von 1 Hz gemäß dem hierin beschriebenen Verfahren, aufweist, der nicht kleiner als 0,1 MPa ist.

22. Verbundstruktur, umfassend:
ein erstes Substrat
ein zweites Substrat
eine Schmelzklebstoffzubereitung nach einem der vorangehenden Ansprüche 1 bis 21, die das erste und das zweite Substrat verbindet, die, wenn beaufschlagt mit einem Flächengewicht zwischen 0,5 g/m² und 50 g/m², der Verbundstruktur eine Zugfestigkeit von größer als 0,25 N pro 50 mm Breite gibt.

23. Hygieneabsorbergegenstand, umfassend die Schmelzklebstoffzubereitung nach einem der vorangehenden Ansprüche 1 bis 21.

24. Absorberhygienegegenstand, umfassend eine Verbundstruktur nach Anspruch 22.

25. Gegenstand nach Anspruch 23 oder 24, **dadurch gekennzeichnet, dass** der Gegenstand eine Babywindel, eine Trainingshosenwindel, eine Windel für inkontinente Erwachsene oder ein Frauenmenstruationskissen ist.

26. Absorberhygienegegenstand nach einem der vorangehenden Ansprüche 23 bis 25, **dadurch gekennzeichnet, dass** die Schmelzklebstoffzubereitung verwendet wird für wenigstens eine der folgenden Verwendungen: i) allgemeiner Konstruktionsklebstoff des gesamten Gegenstands; ii) zum Binden von elastischen Komponenten (Fäden, Schleifen, Folien oder elastische Platten, etc.); iii) zum Festigen und Sichern, sogar bei Verwendung, der Integrität des Absorberkerns des Absorberhygienegegenstands; iv) für das Binden von perforierten Folien sowohl mit einer zweidimensionalen als auch dreidimensionalen Struktur; v) für das Binden eines Vlieses mit einem anderen Vlies oder mit einer Kunststofffolie.

27. Gegenstand umfassend die Schmelzklebstoffzubereitung nach einem der vorangehenden Ansprüche 1 bis 21, **dadurch gekennzeichnet, dass** der Gegenstand eine chirurgische Absorbermatratze oder -bogen oder chirurgisches Laminat zur medizinische Verwendung oder ein Wundabdeckungsprodukt ist.

28. Gegenstand, umfassend die Schmelzklebstoffzubereitung nach einem der vorangehenden Ansprüche 1) bis 21), **dadurch gekennzeichnet, dass** der Gegenstand eine Matratze oder eine Komponente derselben ist.

29. Gegenstand umfassend die Schmelzklebstoffzubereitung nach einem der vorangehenden Ansprüche 1 bis 21, **dadurch gekennzeichnet, dass** der Gegenstand eine Verpackung ist.

30. Gegenstand nach einem der vorangehenden Ansprüche 23 bis 29, **dadurch gekennzeichnet, dass** die Schmelzklebstoffzubereitung in wenigstens einer Anwendung durch das Verfahren einer Schlitzdüsenbeschichtung aufgetragen wird.

## Revendications

1. Formulation d'adhésif thermofusible, qui comprend au moins un polymère, qui peut être un homopolymère ou un copolymère, ou un mélange compatible de polymères, qui peuvent être des homopolymères ou des copolymères, dans laquelle ledit polymère ou mélange compatible de polymères comprend un homopolymère d'oléfines en C2 à C12 ou un copolymère parmi les mêmes oléfines, **caractérisée en ce que** ledit polymère ou mélange compatible de polymères :
A) présente une cinétique de cristallisation qui n'est pas affectée par la pré-application d'une contrainte de cisaillement, selon l'essai de « cristallisation induite sous contrainte de cisaillement », tel que décrit ici ;
B) présente un indice de polydispersité qui n'est pas inférieur à 2,0 et de préférence pas inférieur à 2,5 ;
ladite formulation d'adhésif thermofusible étant en outre **caractérisée en ce qu'**elle présente :
- une viscosité Brookfield à 170 °C, mesurée selon la méthode ASTM D 3236 - 88, dans une plage comprise entre 500 mPa.s et 50 000 mPa.s
- une température de ramollissement anneau et bille, mesurée selon la méthode ASTM D 36 - 95, pas supérieure à 150 °C
- un module élastique G', mesuré à 23 °C et à une fréquence de 1 Hz, qui n'est pas inférieur à 0,05 MPa.

2. Formulation d'adhésif thermofusible comme en 1), **caractérisée en ce que** le polymère ou le mélange compatible de polymères, lorsqu'il est testé dans un essai de fusion DSC, après 24 heures de vieillissement dans des conditions ambiantes, affiche un pic dont la température finale la plus élevée de fin de fusion n'est pas inférieure à 70 °C.

3. Formulation d'adhésif thermofusible selon l'une des revendications précédentes 1) et 2), qui, lorsqu'elle est testée dans un essai de fusion DSC, après vieillissement pendant cinq jours à conditions ambiantes, affiche une enthalpie de fusion qui n'est pas supérieure à 40 J/g.

4. Formulation d'adhésif thermofusible selon l'une des revendications précédentes 1) à 3), dans laquelle le polymère ou le mélange compatible de polymères constitue de 10 % en poids à 99,5 % en poids de ladite formulation d'adhésif thermofusible, et de préférence de 20 % en poids à 90 % en poids.

5. Formulation d'adhésif thermofusible selon l'une des revendications précédentes 1) à 4), qui comprend au moins un agent collant ayant un point de ramollissement anneau et bille, mesuré selon la méthode ASTM D 36 - 95, dans une plage allant de 5 °C à 160 °C.

6. Formulation d'adhésif thermofusible comme en 5), **caractérisée en ce que** l'agent collant ou le mélange d'agents collants est choisi parmi des agents collants hydrocarbonés aliphatiques et leurs dérivés entièrement ou partiellement hydrogénés ; des agents collants hydrocarbonés aromatiques et leurs dérivés entièrement ou partiellement hydrogénés ; des agents collants hydrocarbonés aliphatiques/aromatiques et leurs dérivés entièrement ou partiellement hydrogénés ; des agents collants terpène et agents collants terpène modifiés et leurs dérivés entièrement ou partiellement hydrogénés ; des rosines, leurs esters et leurs dérivés entièrement ou partiellement hydrogénés, et leurs mélanges.

7. Formulation d'adhésif thermofusible comme en 5) ou 6), **caractérisée en ce que** l'agent collant ou le mélange d'agents collants présente un point de ramollissement anneau et bille, mesuré selon la méthode ASTM D 36 - 95, entre 70 °C et 135 °C, de préférence entre 80 °C et 125 °C.

8. Formulation d'adhésif thermofusible selon l'une des revendications précédentes 5) à 7), **caractérisée en ce que** l'agent collant ou le mélange d'agents collants a une teneur en impuretés volatiles qui n'est pas supérieure à 5 ppm et de préférence pas supérieure à 1 ppm.

9. Formulation d'adhésif thermofusible selon l'une des revendications précédentes 5) à 8), **caractérisée en ce que** l'agent collant ou le mélange d'agents collants constitue zéro à 75 % en poids de la formulation d'adhésif, de préférence 10 % en poids à 70 % en poids et de manière davantage préférée 20 % en poids à 65 % en poids.

10. Formulation d'adhésif thermofusible selon l'une des revendications précédentes 1) à 9), qui comprend au moins un plastifiant, qui est liquide à température ambiante, ou un mélange de plastifiants, qui est liquide à température ambiante.

11. Formulation d'adhésif thermofusible comme en 10), **caractérisée en ce que** le plastifiant, qui est liquide à température ambiante, ou le mélange de plastifiants, qui est liquide à température ambiante, est sélectionné parmi des huiles minérales paraffiniques ; des huiles minérales naphténiques ; des hydrocarbures paraffiniques et naphténiques qui sont liquides à température ambiante, et des mélanges de ceux-ci ; des oligomères qui sont liquides à température ambiante de polyoléfines et leurs copolymères, tels que des oligomères liquides dérivés d'éthylène, de propylène, de butène, d'isobutylène, des copolymères de ceux-ci et similaires ; des plastifiants qui sont liquides à température ambiante, formés par des esters, tels que des phtalates, des benzoates, des sébacates ; des huiles végétales ; des graisses naturelles et synthétiques ; et des mélanges de ceux-ci ;

12. Formulation d'adhésif thermofusible comme en 10) et en 11), **caractérisée en ce que** le plastifiant, qui est liquide à température ambiante, ou le mélange de plastifiants, qui est liquide à température ambiante, est formé par un oligomère ou par un mélange d'oligomères de poly-alpha-oléfines synthétiques, synthétisées à partir d'oléfines en C2 à C20 et ayant un poids moléculaire moyen en nombre compris entre 150 g/mol et 15 000 g/mol.

13. Formulation d'adhésif thermofusible selon l'une des revendications précédentes 10) à 12), **caractérisée en ce que** le plastifiant, qui est liquide à température ambiante, ou le mélange de plastifiants, qui est liquide à température ambiante, constitue de zéro à 50 % en poids de la formulation d'adhésif, de préférence de 5 % en poids à 40 % en poids et de manière davantage préférée de 10 % en poids à 35 % en poids.

14. Formulation d'adhésif thermofusible selon l'une des revendications précédentes 1) à 13), **caractérisée en ce que** le plastifiant, qui est liquide à température ambiante, ou le mélange de plastifiants, comprend au moins une cire ou un mélange de cires, ayant un point de goutte, mesuré selon ASTM D-3954-94, compris entre 40 °C et 170 °C.

15. Formulation d'adhésif thermofusible comme en 14), **caractérisée en ce que** la cire ou le mélange de cires est choisi parmi des cires paraffiniques et notamment des cires synthétisées à partir d'oléfines en C2 à C10, et leurs mélanges ; des cires hydrocarbonées en C12 à C40, également dans leurs dérivés modifiés par des groupes carboxyliques acides ou groupes alcooliques ; des cires copolymères synthétisées à partir d'éthylène et d'anhydride maléique ou à partir de propylène et d'anhydride maléique ; des cires microcristallines ; des cires formées par esters d'acides gras en C12 à C40 ; des cires naturelles comme de la cire d'abeille, de la cire de carnauba, de la cire de montan et similaires ; et leurs mélanges.

16. Formulation d'adhésif thermofusible comme en 14) et en 15), **caractérisée en ce que** la cire ou le mélange de cires constitue de zéro à 20 % en poids de la formulation d'adhésif, de préférence de zéro à 10 % en poids, et de manière davantage préférée de zéro à 5 % en poids.

17. Formulation d'adhésif thermofusible selon l'une des revendications précédentes 1) à 16), qui, après vieillissement pendant cinq jours dans des conditions ambiantes, présente une courbe contrainte/élongation à la rupture, mesurée à 23 °C et à la fréquence de 0,01 Hz selon la méthode décrite ici, qui affiche une contrainte maximale (pic) non inférieure à 0,15 MPa.

18. Formulation d'adhésif thermofusible selon l'une des revendications précédentes 1) à 16), qui, après vieillissement pendant cinq jours dans des conditions ambiantes, présente une courbe contrainte/élongation à la rupture, mesurée à 23 °C et à la fréquence de 0,01 Hz selon la méthode décrite ici, qui affiche une élongation à la rupture non inférieure à 50 %.

19. Formulation d'adhésif thermofusible selon l'une des revendications précédentes 1) à 16), qui, après vieillissement pendant cinq jours dans des conditions ambiantes, présente une courbe contrainte/élongation à la rupture, mesurée à 23 °C et à la fréquence de 0,01 Hz selon la méthode décrite ici, qui affiche une ténacité non inférieure à 0,2 MJ/m³.

20. Formulation d'adhésif thermofusible selon l'une des revendications précédentes 1) à 19), qui présente un module élastique G', mesuré à 23 °C et à la fréquence de 1 Hz selon la méthode décrite ici, qui n'est pas inférieur à 0,5 MPa.

21. Formulation d'adhésif thermofusible selon l'une des revendications précédentes 1) à 19), qui présente un module élastique G', mesuré à 37 °C et à la fréquence de 1 Hz selon la méthode décrite ici, qui n'est pas inférieur à 0,1 MPa.

22. Structure liée comprenant :
- un premier substrat
- un second substrat
- une formulation d'adhésif thermofusible selon l'une des revendications précédentes 1) à 21), liant le premier et le second substrat, qui, lorsqu'elle est appliquée à un poids de base compris entre 0,5 g/m² et 50 g/m², confère à la structure liée une résistance au pelage supérieure à 0,25 N par 50 mm de largeur.

23. Article d'hygiène absorbant, comprenant la formulation d'adhésif thermofusible selon l'une des revendications précédentes 1) à 21).

24. Article d'hygiène absorbant, comprenant une structure liée comme en 22).

25. Article comme en 23) ou 24), **caractérisé en ce que** ledit article est une couche pour bébé, une couche de type culotte de propreté, une couche pour adultes incontinents, une serviette hygiénique féminine ;

26. Article d'hygiène absorbant selon l'une des revendications précédentes 23) à 25), **caractérisé en ce que** la formulation d'adhésif thermofusible est utilisée pour au moins l'une des utilisations suivantes :
i) adhésif de construction général de l'article entier ;
ii) pour la liaison de composants élastiques (fils, rubans, films ou panneaux élastiques, etc.) ; iii) pour le renforcement et la garantie, même lors de l'utilisation, de l'intégrité du noyau absorbant de l'article d'hygiène absorbant ; iv) pour la liaison de films perforés à la fois avec une structure bidimensionnelle ou tridimensionnelle ; v) pour la liaison d'un non-tissé à un autre non-tissé ou à un film plastique.

27. Article comprenant la formulation d'adhésif thermofusible selon l'une des revendications précédentes 1) à 21), **caractérisé en ce que** ledit article est un matelas ou drap chirurgical absorbant ou un stratifié chirurgical à usage médical ou un produit de type pansement.

28. Article comprenant la formulation d'adhésif thermofusible selon l'une des revendications précédentes 1) à 21), **caractérisé en ce que** ledit article est un matelas ou un composant de celui-ci.

29. Article comprenant la formulation d'adhésif thermofusible selon l'une des revendications précédentes 1) à 21), **caractérisé en ce que** ledit article est un emballage.

30. Article selon l'une des revendications précédentes 23) à 29), **caractérisé en ce que** la formulation d'adhésif thermofusible est appliquée dans au moins une utilisation par le procédé de revêtement à filière plate.
